Europäisches Patentamt

European Patent Office

Office européen des brevets

(11) Veröffentlichungsnummer: **0 226 164 B1**

(12) **EUROPÄISCHE PATENTSCHRIFT**

(45) Veröffentlichungstag der Patentschrift: **01.04.92**

(21) Anmeldenummer: **86117086.8**

(22) Anmeldetag: **08.12.86**

(51) Int. Cl.⁵: **A61N 1/365**

(54) **Herzschrittmacher.**

(30) Priorität: **20.12.85 DE 3545359**

(43) Veröffentlichungstag der Anmeldung:
**24.06.87 Patentblatt 87/26**

(45) Bekanntmachung des Hinweises auf die
Patenterteilung:
**01.04.92 Patentblatt 92/14**

(84) Benannte Vertragsstaaten:
**DE FR GB IT NL SE**

(56) Entgegenhaltungen:
EP-A- 0 135 911     EP-A- 0 151 689
DE-A- 3 104 938     US-A- 3 815 611
US-A- 4 365 636     US-A- 4 428 378

(73) Patentinhaber: **SIEMENS AKTIENGESELL-
SCHAFT
Wittelsbacherplatz 2
W-8000 München 2(DE)**

(84) Benannte Vertragsstaaten:
**DE FR GB IT NL**

(73) Patentinhaber: **Siemens Elema AB
Röntgenvägen 2
S-171 95 Solna 1(SE)**

(84) Benannte Vertragsstaaten:
**SE**

(72) Erfinder: **Strandberg, Hans
Landsvägen 51
S-172 36 Sundyberg(SE)**
Erfinder: **Hedberg, Sven-Erik
Odonstigen 5
S-196 31 Kungsängen(SE)**
Erfinder: **Obel, Martin
Bergavägen 5
S-182 33 Danderyd(SE)**

EP 0 226 164 B1

## Beschreibung

Die Erfindung bezieht sich auf einen Herzschrittmacher gemäß Oberbegriff des Patentanspruchs 1.

Herzschrittmacher dieser Art sind z.B. durch die US-PS 35 93 718 und die europäische Patentanmeldung 00 89 014 vorbekannt. Die bekannten Herzschrittmacher umfassen einen Impedanzpneumographen zur Erfassung eines Atemsignales, der zusätzliche Elektroden und Energie zur Impedanzmessung benötigt.

Aufgabe vorliegender Erfindung ist es, einen Herzschrittmacher der eingangs genannten Art aufzubauen, dessen Atemsignalmeßeinrichtung das Atemsignal in technisch einfacherer Weise und energieärmer als bisher erfaßt.

Die Aufgabe wird mit den kennzeichnenden Merkmalen des Patentanspruchs 1 gelöst.

Gemäß der Erfindung kann das Atemsignal direkt aus Schwankungen des Herzaktionssignales erfaßt werden. Ein zusätzlicher Strom für eine Impedanzmessung ist nicht erforderlich. Der Herzschrittmacher ist also technisch einfacher und benötigt weniger Energie.

Weitere Vorteile und Einzelheiten der Erfindung ergeben sich aus der nachfolgenden Beschreibung eines Ausführungsbeispiels anhand der Zeichnung in Verbindung mit den Unteransprüchen.

Es zeigen:

Figur 1 in schematischer Darstellung Herzaktionssignale und deren Amplitudenschwankungen, aus denen ein Atemsignal abgeleitet werden kann,
Figur 2 das Meßprinzip anhand eines vergrößerten Ausschnittes der Herzaktionssignale und
Figur 3 die Erfindung im Prinzipschaltbild.

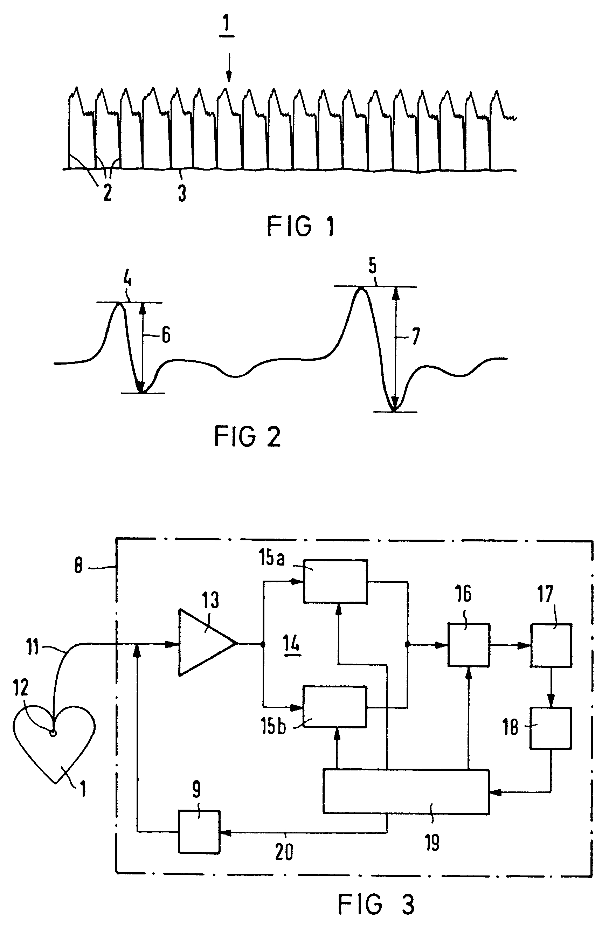

Die Figur 1 zeigt die von einem Herzschrittmacher mittels eines QRS-Detektors erfaßten Herzaktionssignale 1, deren Amplituden 2 (R-Zacken) Schwankungen im Takt der Atmung des Herzschrittmacherträgers unterworfen sind. Die Hüllkurve aller Schwankungen ist mit 3 angedeutet. Sie entspricht gemäß der Erfindung dem Atemsignal.

Die Figur 2 zeigt zwei aufeinanderfolgende QRS-Komplexe 4 und 5 mit unterschiedlichen Amplituden. Wie in der Figur 2 dargestellt, soll jeweils von positiver zu negativer Spitze der R-Zacke gemessen werden, wobei die Abstände 6, 7 zwischen den jeweiligen Spitzenwerten das Maß für das Atemsignal abgeben.

Die Figur 3 zeigt einen in einem Gehäuse 8 eingeschlossenen Herzschrittmacher mit einem Pulsgenerator 9 für die Herzschrittmacherpulse, die dem Herz 10 des Herzschrittmacherträgers über eine Elektrodenleitung 11 mit im Herzen 10 eingesetzter Herzschrittmacherelektrode 12 zugeleitet werden. Der Herzschrittmacher der Figur 3 umfaßt ferner einen Verstärker 13 für über die Elektrodenleitung 11 erfaßte Herzaktionssignale. Auf den Verstärker 13 folgt eine Spitzenabtasteinrichtung 14 für QRS-Komplexe mit einem ersten Sample & Hold-Glied 15a für positive Spitzen und einem zweiten Sample & Hold-Glied 15b für negative Spitzen.

Die erfaßten Spitzenwerte werden mittels Multiplexer 16 und nach Analog-Digital-Umwandlung in einem A/D-Wandler 17 einem Differenzbildner 18 zugeleitet, der die Differenzabstände zwischen den erfaßten positiven und negativen Spitzen eines jeden QRS-Komplexes erfaßt. Das Ausgangssignal des Differenzbildners 18 wird als Atemsignal der Steuerelektronik 19 des Herzschrittmachers zugeführt. Die Steuerelektronik 19 steuert über eine Steuerleitung 20 den Pulsgenerator 9 in dem Sinne, daß mit zunehmender Frequenz des Atemsignales am Ausgang des Differenzbildners 18 die Pulsfolgefrequenz der Schrittmacherpulse von einem Grundwert entsprechend zu höheren Werten verschoben wird. Damit wird auf einfache Weise die Schrittmacherfrequenz an den erhöhten Bedarf des Schrittmacherträgers entsprechend angepaßt.

## Patentansprüche

1. Herzschrittmacher mit einem Pulsgenerator für die Schrittmacherpulse, einer Atemsignalmeßeinrichtung zum Erfassen eines Atemsignales und einer Steuereinrichtung zum Steuern des Pulsgenerators im Sinne der Änderung der Pulsfolgefrequenz des Pulsgenerators in Abhängigkeit von der Frequenz des Atemsignals, **dadurch gekennzeichnet,** daß ein Herzaktionsdetektor (13) zum Erfassen von Herzaktionssignalen vorhanden ist und daß die Atemsignalmeßeinrichtung eine Vorrichtung (14 bis 18) umfaßt, die Amplitudenschwankungen im Herzaktionssignal erfaßt und als Atemsignal der Steuereinrichtung (19) für den Pulsgenerator (9) zuführt.

2. Herzschrittmacher nach Anspruch 1, **dadurch gekennzeichnet,** daß die Atemsignalmeßeinrichtung einen Spitzenwertabtaster (15a,15b) für die positiven und negativen Spitzen der Herzaktionssignale umfaßt und daß dem Spitzenwertabtaster ein Differenzbildner (18) zur Ermittlung des Abstandes zwischen positiven und negativen Spitzen nachgeschaltet ist und daß die so erfaßten Abstände das Maß für das Atemsignal abgeben.

## Claims

1. Heart pacemaker having a pulse generator for the pacemaker pulses, a respiration signal measuring device for detecting a respiration signal and a control device for controlling the pulse generator for changing the pulse sequence frequency of the pulse generator as a function of the frequency of the respiration signal, characterised in that a heart action detector (13) for detecting heart action signals is present, and in that the respiration signal measuring device comprises a device (14 to 18) which detects amplitude fluctuations in the heart action signal and feeds them as a respiration signal to the control device (19) for the pulse generator (9).

2. Heart pacemaker according to Claim 1, characterised in that the respiration signal measuring device comprises a peak value sensor (15a, 15b) for the positive and negative peaks of the heart action signals, and in that a difference former (18) for determining the distance between positive and negative peaks is connected downstream of the peak value sensor, and in that the distances detected in this way supply the dimension for the respiration signal.

**Revendications**

1. Stimulateur cardiaque comportant un générateur d'impulsions servant à délivrer les impulsions de stimulation cardiaque, un dispositif de mesure d'un signal de respiration servant à détecter un signal de respiration, et un dispositif de commande servant à commander le générateur d'impulsions en vue de modifier la fréquence de récurrence des impulsions de ce générateur en fonction de la fréquence du signal de respiration, caractérisé par le fait qu'il est prévu un détecteur (13) de l'activité cardiaque servant à détecter des signaux d'activité cardiaque, et que le dispositif de mesure du signal de respiration comprend un dispositif (14 à 18), qui détecte des variations d'amplitude du signal d'activité cardiaque et les envoie en tant que signal de respiration au dispositif (19) qui sert à commander le générateur d'impulsions (9).

2. Stimulateur cardiaque suivant la revendication 1, caractérisé par le fait que le dispositif de mesure du signal de respiration comprend un dispositif (15a,15b) servant à échantillonner les pointes positives et négatives des signaux d'activité cardiaque et qu'en aval du dispositif d'échantillonnage des valeurs de pointes est branché un circuit de formation de différence (18) servant à déterminer l'écart entre les pointes positive et négative, et que les intervalles ainsi détectés fournissent la mesure pour le signal de respiration.

FIG 1

FIG 2

FIG 3